# EUROPEAN PATENT APPLICATION

(11) **EP 1 709 961 A1**
(43) Date of publication of application: **11.10.2006**
(21) Application number: 05255578.6
(22) Date of filing: 12.09.2005
(51) Int. Cl.: A61K 31/202, A61P 11/00, A23L 1/29, A23L 1/30

(54) **Use of Docosahexaenoic Acid and/or Arachidonic Acid for preventing or treating respiratory infections in infants**

(30) Priority: 07.04.2005 US 669111 P; 26.04.2005 US 114892
(71) Applicant: Bristol-Myers Squibb Company, Princeton, New Jersey 08543-4000 (US)
(72) Inventor: Lifschitz, Carlos H., Houston, TX 77005 (US); Pastor, Nitida, 28040 Madrid (ES)
(74) Representative: Adams, Harvey Vaughan John

(57) **Abstract**

The present invention is directed to a novel method for preventing or treating respiratory infections in infants. The method comprises administration of a therapeutically effective amount of DHA and ARA to the infant.

## Description

### BACKGROUND OF THE INVENTION

This application claims priority back to United States Provisional Application 60/669,111 filed April 7, 2005, which is incorporated by reference herein in its entirety.

### (1) Field of the Invention

The present invention relates generally to a method for preventing or treating respiratory infections in infants.

### (2) Description of the Related Art

The respiratory tract is the passageway for air between the outer atmosphere and the alveoli, which are tiny air sacs of the lungs. The respiratory tract is divided into the upper and lower respiratory tract. The upper respiratory tract includes the nose, throat, larynx and upper trachea. The lower respiratory tract includes the bronchi, bronchioles and lung substance.

Respiratory tract infections are extremely common, especially in infants. In the first year of life, infants are prone to recurrent respiratory tract infections, often experiencing between three and six infections during that year alone.

Respiratory infections and their symptoms can range from mild to severe, depending on the type of virus and the location of the infection. Upper respiratory infections often manifest themselves as common colds, causing inflammation and swelling of the lining of the nose, throat and sinuses. Influenza, commonly known as the flu, is a highly contagious viral infection of the upper respiratory tract. Symptoms of the flu include fever, chills, headache, muscle aches, dizziness, cough, sore throat, runny nose, nausea and diarrhea. Another upper respiratory infection, croup, causes a very deep cough and varying degrees of breathing difficulty, primarily when inhaling.

Lower respiratory infections are generally considered more serious than upper respiratory infections. Respiratory syncytial virus (RSV) is the most frequent cause of lower respiratory tract infections in infants and children younger than 4 years of age. Van Woensel, J., et al., Viral Lower Respiratory Tract Infection in Infants and Young Children, BMJ 327:36-40 (2003). This is such a common virus that virtually all children have been infected with RSV by the age of 3. In most infants and children, RSV is a mild respiratory infection that is indistinguishable from a common cold. It usually causes nasal stuffiness and discharge, cough and sometimes acute otitis media, or middle ear infection. Life threatening apnea could occur in very young infants infected with RSV.

Bronchitis is a lower respiratory infection that affects the brochial tubes, causing narrowing and swelling due to viral inflammation. Bronchiolitis is similar to bronchitis, but occurs primarily in infants. It is an inflammation of the smaller caliber tubes of the branching network of brochi. The infection causes labored breathing, frequent and dramatic coughing and wheezing and may require hospitalization.

The lower respiratory infection that causes the most anxiety for parents is pneumonia in infants. This is caused by an infection in the alveoli, causing them to become filled with fluid, often of a thick purulent nature, which interferes with proper exchange of carbon dioxide. The severity of the pneumonia will depend on the amount of lung tissue involved.

Most upper and lower respiratory infections are caused by viruses for which no specific prevention or treatment is currently available. Some respiratory infections, including influenza, may be prevented with a vaccination. However, even when vaccinations are developed for specific respiratory infections, they are expensive and not universally available. Similarly, drugs to treat these infections have limited availability and are expensive. Thus, it would be useful to provide a non-medicinal method for the treatment or prevention of respiratory infections in infants.

### SUMMARY OF THE INVENTION

Briefly, the present invention is directed to a novel method for preventing or treating respiratory infections in infants, the method comprising administering to the infant a therapeutically effective amount of DHA and ARA.

Among the several advantages found to be achieved by the present invention, is that it provides a method for preventing or treating respiratory infections in infants without the necessity of administering unavailable or costly medications or vaccinations.

### BRIEF DESCRIPTION OF THE DRAWINGS

For a more complete understanding of the present invention, reference is now made to the following description taken in conjunction with the accompanying drawing.

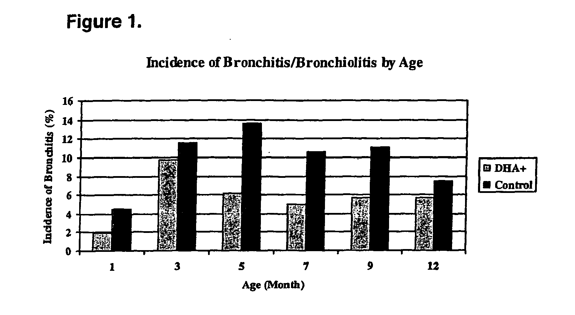

Figure 1 illustrates the effect of DHA and ARA supplementation on the incidence of bronchitis and bronchiolitis in infants between the ages of 1 and 12 months.

### DETAILED DESCRIPTION OF THE PREFERRED EMBODIMENTS

Reference now will be made in detail to the embodiments of the invention, one or more examples of which are set forth below. Each example is provided by way of explanation of the invention, not a limitation of the invention. In fact, it will be apparent to those skilled in the art that various modifications and variations can be made in the present invention without departing from the scope or spirit of the invention. For instance, features illustrated or described as part of one embodiment, can be used on another embodiment to yield a still further embodiment.

Thus, it is intended that the present invention covers such modifications and variations as come within the scope of the appended claims and their equivalents. Other objects, features and aspects of the present invention are disclosed in or are obvious from the following detailed description. It is to be understood by one of ordinary skill in the art that the present discussion is a description of exemplary embodiments only, and is not intended as limiting the broader aspects of the present invention.

### Definitions

As used herein, the term "treating" means ameliorating, improving or remedying a disease, disorder, or symptom of a disease or condition.

The term "preventing" means stopping or hindering a disease, disorder, or symptom of a disease or condition through some action.

The terms "therapeutically effective amount" refer to an amount that results in an improvement or remediation of the disease, disorder, or symptoms of the disease or condition.

The term "infant" means a human that is less than about 1 year old.

The terms "respiratory infection" or "respiratory illness" mean a disease or infection affecting the group of organs responsible for carrying oxygen from the air to the bloodstream and for expelling carbon dioxide.

As used herein, the term "infant formula" means a composition that satisfies the nutrient requirements of an infant by being a substitute for human milk. In the United States, the contents of an infant formula are dictated by the federal regulations set forth at 21 C.F.R. Sections 100, 106, and 107. These regulations define macronutrient, vitamin, mineral, and other ingredient levels in an effort to stimulate the nutritional and other properties of human breast milk.

### Invention

In accordance with the present invention, a novel method for preventing or treating respiratory infections in infants has been discovered. The method comprises administering a therapeutically effective amount of DHA and ARA to the infant.

DHA and ARA are long chain polyunsaturated fatty acids (LCPUFA) which have been shown to contribute to the health and growth of infants. Specifically, DHA and ARA have been shown to support the development and maintenance of the brain, eyes and nerves of infants. Birch, E., et al., A Randomized Controlled Trial of Long-Chain Polyunsaturated Fatty Acid Supplementation of Formula in Term Infants after Weaning at 6 Weeks of Age, Am. J. Clin. Nutr. 75:570-580 (2002). Clandinin, M., et al., Formulas with Docosahexaenoic Acid (DHA) and Arachidonic Acid (ARA) Promote Better Growth and Development Scores in Very-Low-Birth- Weight Infants (VLBW), Pediatr. Res.51:187A-188A (2002). DHA and ARA are typically obtained through breast-milk in infants that are breast-fed. In infants that are formula-fed, however, DHA and ARA must be supplemented into the diet.

While it has been shown that DHA and ARA are beneficial to the development of brain, eyes and nerves in infants, DHA and ARA have not previously been shown to have any effect on respiratory infections. The positive effects of DHA and ARA on respiratory infections that were discovered in the present invention were surprising and unexpected, as breast milk, which contains DHA and ARA, is still not conclusively beneficial for preventing or treating respiratory infections. Among studies that focus on the effect of breastfeeding on respiratory infection, some have found a protective role for breastfeeding, whereas some have not. Sinha, A., et al., Reduced Risk of Neonatal Respiratory Infections Among Breastfed Girls but Not Boys, Pediatr. 112:303-307 (2003).

One study has shown that fish oil, which contains DHA and eicosapentaenoic acid (EPA), can reduce the risk of current asthma, which was defined as wheeze plus exercise-induced airway hyperresponsiveness (AHR). Hodge, L., et al., Consumption of Oily Fish and Childhood Asthma Risk, Med. J. Austral. 164:137-140 (1996). Another study concluded that dietary supplementation with fish oil rich in EPA and DHA for 10 months decreases asthma scores and increases acetylcholine thresholds in children with bronchial asthma. Nagakura, T., et al., Dietary Supplementation with Fish Oil Rich in ω-3 Polyunsaturated Fatty Acids in Children with Bronchial Asthma, Eur. Respir. J. 16:861-865 (2000). These studies, however, do not disclose or suggest the surprising treatment and preventative effects that DHA and ARA have on respiratory infections in infants.

In the present invention, the form of administration of DHA and ARA is not critical, as long as a therapeutically effective amount is administered to the infant. Most conveniently, DHA and ARA can be supplemented into infant formula which can then be fed to an infant.

In an embodiment, the infant formula for use in the present invention is nutritionally complete and contains suitable types and amounts of lipid, carbohydrate, protein, vitamins and minerals. The amount of lipid or fat typically can vary from about 3 to about 7 g/100 kcal. The amount of protein typically can vary from about 1 to about 5 g/100 kcal. The amount of carbohydrate typically can vary from about 8 to about 12 g/100 kcal. Protein sources can be any used in the art, e.g., nonfat milk, whey protein, casein, soy protein, hydrolyzed protein, partially hydrolyzed protein, amino acids, and the like. Carbohydrate sources can be any used in the art, e.g., lactose, glucose, com syrup solids, maltodextrins, sucrose, starch, rice syrup solids, and the like. Lipid sources can be any used in the art, e.g., vegetable oils such as palm oil, soybean oil, palmolein, coconut oil, medium chain triglyceride oil, high oleic sunflower oil, high oleic safflower oil, and the like.

Conveniently, commercially available infant formula can be used. For example, Enfalac, Enfamil®, Enfamil® Premature Formula, Enfamil® with Iron, Lactofree®, Nutramigen®, Pregestimil®, and ProSobee® (available from Mead Johnson & Company, Evansville, IN, U.S.A.) may be supplemented with suitable levels of DHA and ARA and used in practice of the method of the invention. Additionally, Enfamil® LIPIL®, which contains effective levels of DHA and ARA, is commercially available and may be utilized in the present invention.

As an alternative to an infant formula, the DHA and ARA can be administered as a supplement not integral to the formula feeding. For example, the DHA and ARA can be administered as oil drops or sachets or in combination with other nutrient supplements such as vitamins, and the like.

The method of the invention requires the administration of a combination of DHA and ARA. In this embodiment, the weight ratio of ARA:DHA is typically from about 1:3 to about 9:1. In one embodiment of the present invention, this ratio is from about 1:2 to about 4:1. In yet another embodiment, the ratio is from about 2:3 to about 2:1. In one particular embodiment the ratio is about 2:1.

The effective amount of DHA in an embodiment of the present invention is typically from about 3 mg per kg of body weight per day to about 150 mg per kg of body weight per day. In one embodiment of the invention, the amount is from about 6 mg per kg of body weight per day to about 100 mg per kg of body weight per day. In another embodiment the amount is from about 10 mg per kg of body weight per day to about 60 mg per kg of body weight per day. In yet another embodiment the amount is from about 15 mg per kg of body weight per day to about 30 mg per kg of body weight per day.

The effective amount of ARA in an embodiment of the present invention is typically from about 5 mg per kg of body weight per day to about 150 mg per kg of body weight per day. In one embodiment of this invention, the amount varies from about 10 mg per kg of body weight per day to about 120 mg per kg of body weight per day. In another embodiment, the amount varies from about 15 mg per kg of body weight per day to about 90 mg per kg of body weight per day. In yet another embodiment, the amount varies from about 20 mg per kg of body weight per day to about 60 mg per kg of body weight per day.

The amount of DHA in infant formulas for use in the present invention typically varies from about 2 mg/100 kilocalories (kcal) to about 50 mg/100 kcal. In another embodiment, the amount of DHA varies from about 5 mg/100 kcal to about 33 mg/100 kcal. In yet another embodiment, the amount of DHA varies from about 10 mg/100 kcal to about 25 mg/100 kcal. In a particular embodiment, the amount of DHS varies from about 15 mg/100 kcal to about 20 mg/100 kcal.

The amount of ARA in infant formulas for use in the present invention typically varies from about 4 mg/100 kilocalories (kcal) to about 100 mg/100 kcal. In another embodiment, the amount of ARA varies from about 10 mg/100 kcal to about 67 mg/100 kcal. In yet another embodiment, the amount of ARA varies from about 20 mg/100 kcal to about 50 mg/100 kcal. In a particular embodiment, the amount of ARA varies from about 30 mg/100 kcal to about 40 mg/100 kcal.

The infant formula supplemented with oils containing DHA and ARA for use in the present invention can be made using standard techniques known in the art. For example, replacing an equivalent amount of an oil normally present, e.g., high oleic sunflower oil.

The source of the ARA and DHA can be any source known in the art such as fish oil, single cell oil, egg yolk lipid, brain lipid, and the like. The DHA and ARA can be in natural form, provided that the remainder of the LC PUFA source does not result in any substantial deleterious effect on the infant. Alternatively, the DHA and ARA can be used in refined form. It is preferred that the LCPUFA used in the invention contain little or no EPA. For example, it is preferred that the infant formulas used herein contain less than about 20 mg/100 kcal EPA; preferably less than about 10 mg/100 kcal EPA; more preferably less than about 5 mg/100 kcal EPA; and most preferably substantially no EPA.

Sources of DHA and ARA as single cell oils are taught in U.S. Pat. Nos. 5,374,657, 5,550,156, and 5,397,591, the disclosures of which are incorporated herein by reference in their entirety.

In an embodiment of the present invention, the DHA and ARA are supplemented into the diet of the infant from birth until the infant reaches about one year of age. In another embodiment, DHA and ARA are administered prenatally to the mother and/or supplemented into the postnatal diet of the infant until the infant reaches about one year of age.

In a separate embodiment, a method is provided for preventing or treating respiratory infections in infants, the method comprising administering to the infant a therapeutically effective amount of DHA. In yet another embodiment, a method is provided for preventing or treating respiratory infections in infants, the method comprising administering to the infant a therapeutically effective amount of ARA. It is believed that the administration of DHA or ARA alone may prevent or treat respiratory infections in infants.

In an embodiment, DHA- and ARA-supplementation prevent or treat the occurrence of upper respiratory infection, influenza, croup, respiratory syncytial virus, bronchitis, bronchiolitis and/or pneumonia. As will be seen in the examples, DHA and ARA have been shown to specifically reduce the incidence of bronchitis/bronchiolitis, upper respiratory infection and overall respiratory infections.

The following examples describe various embodiments of the present invention. Other embodiments within the scope of the claims herein will be apparent to one skilled in the art from consideration of the specification or practice of the invention as disclosed herein. It is intended that the specification, together with the examples, be considered to be exemplary only, with the scope and spirit of the invention being indicated by the claims which follow the examples. In the examples, all percentages are given on a weight basis unless otherwise indicated.

### Example 1

This example describes the materials and methods necessary to show the effect of administration of a DHA- and ARA-supplemented infant formula on respiratory infections in infants. A study was conducted to assess and compare the frequency of the appearance of intercurrent illnesses in a long-term follow-up between two groups of patients receiving artificial feeding with or without DHA and ARA. As secondary objectives, weight, height, and head circumference were measured. Additionally, acceptability and tolerance of the artificial feed were evaluated.

The study was a multicenter, prospective, observation study conducted in Spain with a 12-month long-term follow-up. The study included a total of 1,345 children, recruited by 357 pediatricians whose geographic distribution assured that the entire country was represented. The children were selected without following any randomization criteria. They were assigned to the artificial nursing formula that the pediatrician believed to be appropriate for that child.

Infants were assigned to be fed either DHA- (17 mg/100 Kcal) and ARA- (34 mg/100 Kcal) supplemented formula (DHA/ARA) (Enfalac Premium®, Mead Johnson Company) or non-supplemented formula (control) for 1 year. A total of 1,094 infants were fed DHA-/ARA-supplemented formula and 248 infants were fed control formula. Three infants were not assigned to any formula. The amount of DHA/ARA that was supplemented into the formula was similar to that found in breast milk. Infants returned at 1, 3, 5, 7, 9, and 12 months of age for follow-up anthropometrics. At each visit, physician records were reviewed for diagnoses of medical illness.

### Example 2

This example illustrates the family history data on the study participants. In summary, the study participants had the following conditions as a part of their family history: asthma (10.9%), allergic rhinitis (1.6%), atopic dermatitis (5.9%), allergy to cow's milk protein (0.5%), other allergies (6.7%), and metabolic illnesses (2.2%). There were no differences in the frequency of family antecedents between the DHA/ARA and control groups, resulting in a homogenous population.

The mean age for beginning formula feeding was 1.16 months (Confidence limits (CI) 95% 1.1 to 1.2) with ages between 0 and 4 months. The study participants included 466 newborn infants, 474 infants that were one month old, 199 infants that were two months old, 138 infants that were three months old, and 68 infants that were four months old. The baseline age difference between the two groups studied was 0.2 months, with the DHA/ARA-supplemented infants being older. This difference was not clinically relevant to the study results.

The percentage of males in the study was 54.1 % (719) and the percentage of females was 45.4% (611). In 15 cases, the infants' gender was not specified. The DHA/ARA group and control group were homogeneous concerning the proportion of boys and girls.

The mean gestational period for study infants was 39.1 weeks (Cl 95% 39.1 to 39.2) ranging between 28 and 50 weeks, which were extreme value exceptions as demonstrated by the small mean confidence interval. The DHA/ARA group and control group were homogeneous with regards to gestation period.

The mean activity, pulse, grimace, appearance and respiration (APGAR) test value at birth was 8.95 points (Cl 95% 8.91 to 8.99) ranging between 2 and 10 points. Only four infants had scores below 5. The DHA/ARA group and control group were homogeneous with regards to APGAR scores.

The mean weight at birth for the males was 3,277.4 grams (Cl 95% 3,244.6 to 3,310.5) ranging between 1,440 and 4,690 grams. The mean weight at birth for the females was 3,188.8 grams (Cl 95% 3,155.1 to 3,222.5) ranging between 1,250 and 4,250 grams. The DHA/ARA group and control group were homogeneous with regards to weight.

The mean length at birth for the males was 499.4 mm (Cl 95% 497.7 to 501) ranging between 380 and 570 mm. The mean length at birth for the females was 493.4 mm (Cl 95% 491.9 to 494.95) ranging between 420 and 550 mm. The DHA/ARA group and control group were homogeneous with regards to height at birth.

The mean cranial perimeter at birth for the males was 346.8 mm (CI 95% 345.5 to 348.1) ranging between 300 and 470 mm. The mean cranial perimeter at birth for the females was 342.9 mm (CI 95% 341.6 to 344.3) ranging between 290 and 470 mm. The DHA/ARA group and control group were homogeneous with regards to cranial perimeter at birth.

The most frequent anomaly was dermatological (7.1 %, 96 infants), followed by muscle-skeletal anomalies (5.7%, 76 infants), and genitor-urinary (3.9%, 53 infants). The DHA/ARA group and control group were homogeneous for the frequency of all anomalies found in the basal exploration.

### Example 3

This example illustrates the effects of DHA/ARA-supplementation on various infections and symptoms of infections during the first year of life. Table 1 displays the percentage of DHA/ARA-supplemented infants compared to the percentage of control-fed infants that presented infections or symptoms of infections during their follow-up visits.

**Table 1. DHA/ARA vs. Control - Symptoms of Infection**

| **Symptom** | **Follow-Up Month** | **Control %** | **DHA/ARA %** | **P** |
|---|---|---|---|---|
| Irritability | 1 | 4 | 1.3 | F 0.05 |
| Infection of upper airways | 1 | 12.1 | 6.6 | 0.05 |
| | 12 | 24.2 | 16.2 | 0.01 |
| Rhinitis | 1 | 6.7 | 3 | 0.05 |
| Bronchitis/ brochiolits | 5 | 13.9 | 6.1 | 0.0001 |
| | 7 | 10.8 | 5.1 | 0.01 |
| | 9 | 11.3 | 5.8 | 0.01 |
| Vomiting | 5 | 0 | 2.5 | F 0.05 |
| Constipation | 3 | 9.7 | 5.3 | 0.05 |
| Endocrine | 7 | 1 | 0 | F 0.05 |

F= Results of the Fisher Exact Test. The results are statistically significant if P<0.05.

In all cases, the percentage of infants in the control group that suffered infections or symptoms of infections was higher than in the DHA/ARA group. The only exception was vomiting at month 5.

Table 2 show the results of the study grouped according to the bodily system in which the symptom occurred. Only the results that are statistically significant are shown.

**Table 2. DHA/ARA vs. Control - Grouped According to System**

| **System** | **Follow-up Month** | **Control %** | **DHA/ARA %** | **P** |
|---|---|---|---|---|
| ORL | 1 | 20.1 | 12.6 | 0.05 |
| | 12 | 37.6 | 26.7 | 0.01 |
| Gastrointestinal | 3 | 20 | 11.9 | 0.01 |
| Respiratory | 5 | 16 | 9 | 0.01 |
| | 7 | 12.9 | 7.6 | 0.05 |
| | 9 | 16.5 | 8.9 | 0.01 |
| Endocrine | 7 | 1 | 0 | F 0.05 |

In summary, the percentage of infants in the control group that presented symptoms of bronchitis/bronchiolitis (37.3%) was higher than the percentage of infants in the DHA/ARA group (27.4%) presenting similar symptoms. Both Table 1 and Figure 1 show that at 5, 7 and 9 months of age, the incidence of bronchitis/bronchiolitis was significantly higher in the control-fed group. Additionally, the percentage of infants that developed infection of the upper airways at 12 months of age was higher in the control group than in the DHA/ARA group. The incidence of physician recorded respiratory illness overall was lower in the DHA/ARA group.

Thus, the results of this study show that infants fed DHA/ARA-supplemented formula have a decreased incidence of bronchitis/bronchiolitis, upper respiratory infection and overall respiratory infections during the first year of life when compared to infants fed formula that does not contain DHA and ARA.

All references cited in this specification, including without limitation, all papers, publications, patents, patent applications, presentations, texts, reports, manuscripts, brochures, books, intemet postings, journal articles, periodicals, and the like, are hereby incorporated by reference into this specification in their entireties. The discussion of the references herein is intended merely to summarize the assertions made by their authors and no admission is made that any reference constitutes prior art. Applicants reserve the right to challenge the accuracy and pertinence of the cited references

These and other modifications and variations to the present invention may be practiced by those of ordinary skill in the art, without departing from the spirit and scope of the present invention, which is more particularly set forth in the appended claims. In addition, it should be understood that aspects of the various embodiments may be interchanged in whole or in part. Furthermore, those of ordinary skill in the art will appreciate that the foregoing description is by way of example only, and is not intended to limit the invention so further described in such appended claims. Therefore, the spirit and scope of the appended claims should not be limited to the description of the preferred versions contained therein.

## Claims

1. Use of DHA and ARA in the manufacture of a medicament for the prevention or treatment of a respiratory infection in an infant.

2. The use according to claim 1, wherein the respiratory infection is selected from the group consisting of upper respiratory infection, influenza, croup, respiratory syncytial virus, bronchitis, bronchiolitis and pneumonia.

3. The use according to claim 1, wherein the medicament is to be administered to the infant from the time of birth until about one year of age.

4. The use according to claim 1, wherein the amount of DHA to be administered is between about 3 mg per kg of body weight per day and 150 mg per kg of body weight per day.

5. The use according to claim 1, wherein the amount of DHA to be administered is between about 10 mg per kg of body weight per day and 60 mg per kg of body weight per day.

6. The use according to claim 1, wherein the amount of DHA to be administered is between about 15 mg per kg of body weight per day and 30 mg per kg of body weight per day.

7. The use according to claim 1, wherein the amount of ARA to be administered is between about 5 mg per kg of body weight per day and 150 mg per kg of body weight per day.

8. The use according to claim 1, wherein the amount of ARA to be administered is between about 15 mg per kg of body weight per day and 90 mg per kg of body weight per day.

9. The use according to claim 1, wherein the amount of ARA to be administered is between about 20 mg per kg of body weight per day and 60 mg per kg of body weight per day.

10. The use according to claim 1, wherein the ratio of ARA:DHA in the medicament is from about 1:3 to about 9:1.

11. The use according to claim 1, wherein the ratio of ARA:DHA in the medicament is from about 1:2 to about 4:1.

12. The use according to claim 1, wherein the ratio of ARA:DHA in the medicament is from about 2:3 to about 2:1.

13. The use according to claim 1, wherein the ratio of ARA:DHA in the medicament is about 2:1.

14. The use according to claim 1, wherein the medicament is an infant formula.

15. The use according to claim 14, wherein the infant formula comprises DHA in an amount of from about 2 mg to about 50 mg per 100 kcal infant formula.

16. The use according to claim 14, wherein the infant formula comprises DHA in an amount of from about 5 mg to about 33 mg per 100 kcal infant formula.

17. The use according to claim 14, wherein the infant formula comprises DHA in an amount of from about 15 mg to about 20 mg per 100 kcal infant formula.

18. The use according to claim 14, wherein the infant formula comprises ARA in an amount of from about 4 mg to about 100 mg per 100 kcal infant formula.

19. The use according to claim 14, wherein the infant formula comprises ARA in an amount of from about 10 mg to about 67 mg per 100 kcal infant formula.

20. The use according to claim 14, wherein the infant formula comprises ARA in an amount of from about 30 mg to about 40 mg per 100 kcal infant formula.

21. The use according to claim 1, wherein the prevention or treatment additionally comprises prenatal administration of DHA and ARA to the mother of the infant.

22. Use of DHA and ARA in the manufacture of a medicament for the prevention or treatment of bronchitis in an infant.

23. Use of DHA and ARA in the manufacture of a medicament for the prevention or treatment of bronchiolitis in an infant.

24. Use of DHA and ARA in the manufacture of a medicament for the prevention or treatment of an upper respiratory infection in an infant.

25. Use of ARA in the manufacture of a medicament for the prevention or treatment of a respiratory infection in an infant.

26. The use according to claim 25, wherein said prevention or treatment additionally comprises the administration of DHA to the infant.

27. Use of DHA in the manufacture of a medicament for the prevention or treatment of a respiratory infection in an infant.

28. The use according to claim 27, wherein said prevention or treatment additionally comprises the administration of ARA to the infant.

29. The use according to any of claims 25-28, wherein the respiratory infection is selected from the group consisting of an upper respiratory infection, influenza, croup, respiratory syncytial virus, bronchitis, bronchiolitis and pneumonia.

30. A composition comprising DHA and/or ARA in a therapeutically effective amount for use in the prevention or treatment of a respiratory infection in an infant.

31. The composition according to claim 30, wherein the composition is an infant formula.

32. The composition according to claim 30 or 31, wherein the composition contains less than about 20 mg per 100 kcal EPA, preferably less than about 10 mg per 100 kcal EPA, more preferably less than about 5 mg per 100 kcal EPA, and most preferably is substantially free of EPA.
